(19) Europäisches Patentamt
European Patent Office
Office européen des brevets

(11) Numéro de publication: **0 274 953 B1**

# (12) FASCICULE DE BREVET EUROPEEN

(45) Date de publication du fascicule du brevet: **12.09.90**

(51) Int. Cl.[5]: **C07C 69/96**, C07C 68/06

(21) Numéro de dépôt: **87402878.0**

(22) Date de dépôt: **16.12.87**

(54) **Procédé de préparation de carbonates organiques par transesterification.**

(30) Priorité: **23.12.86 FR 8618057**

(43) Date de publication de la demande:
**20.07.88 Bulletin 88/29**

(45) Mention de la délivrance du brevet:
**12.09.90 Bulletin 90/37**

(84) Etats contractants désignés:
**AT BE CH DE ES FR GB GR IT LI LU NL SE**

(56) Documents cités:
**EP-A- 0 110 629**
**EP-A- 0 150 962**
**JP-A-57 176 932**

(73) Titulaire: **SOCIETE FRANCAISE D'ORGANO-SYNTHESE, 15, boulevard de l'Amiral Bruix, F-75116 Paris(FR)**

(72) Inventeur: **Proux, Yves, 39, rue des Cévennes, F-75015 Paris(FR)**
Inventeur: **Pellegrina, Michel, 14, rue Mignon Boucher, F-95100 Argenteuil(FR)**

(74) Mandataire: **Vignally, Noel et al, RHONE-POULENC INTERSERVICES Service Brevets Chimie Centre de Recherches de Saint-Fons B.P. 62, F-69192 Saint-Fons Cedex(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention a pour objet un procédé de préparation de carbonates organiques par transesterification d'un diester de l'acide carbonique et d'un alcool.

Il est connu de préparer des carbonates de dialkyle par transesterification d'un carbonate d'alkylene ou d'un carbonate de diméthyle par un alcool en présence d'un sel d'un métal alcalin comme catalyseur (brevet américain n° 3.642.858, brevet allemand n° 2.749.754).

La demanderesse a trouvé un procédé permettant de diminuer les quantités de catalyseur basique à mettre en oeuvre et/ou de raccourcir les durées de réaction.

L'invention a pour objet un procédé de préparation de carbonates organiques par transesterification d'un diester de l'acide carbonique et d'au moins un alcool plus lourd que celui ou ceux formés par transesterification, en présence d'un catalyseur de transesterification à base d'un sel ionique basique, <u>caractérisé en ce que</u> ladite opération de transesterification est réalisée en outre en présence d'un composé complexant susceptible de solubiliser au moins partiellement ledit sel dans le milieu réactionnel par complexation de son cation et de dissocier au moins partiellement ledit sel.

Parmi les diesters de l'acide carbonique peuvent être mis en oeuvre de préférence ceux de formule

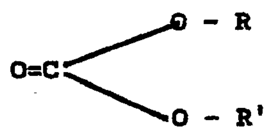

où R et R′ sont identiques ou différents et représent des radicaux alkyles linéaires ou ramifiés en $C_1$-$C_{10}$, de préférence en $C_1$-$C_3$, ou alkenyles linéaires ou ramifiés en $C_3$-$C_{10}$, de préférence en $C_3$-$C_4$.

A titre d'exemples de diesters de l'acide carbonique on peut citer : le carbonate de diméthyle, le carbonate de diethyle, le carbonate dipropyle, le carbonate de méthylethyle, le carbonate de diallyle, le carbonate de methylallyle, le carbonate de propylallyle, le carbonate de dimethallyle...

L'alcool ou les alcools mis en oeuvre sont des monoalcools ou des polyols dont la seule caractéristique est d'être plus lourds que l'alcool ou les alcools formés par transesterification.

Ainsi ledit alcool ou lesdits alcools peuvent être un monoalcool aliphatique saturé ou insaturé, linéaire ou ramifié en $C_1$-$C_{22}$, de préférence en $C_8$-$C_{18}$ ; un monoalcool cycloaliphatique en $C_6$ ; un polyol aliphatique saturé ou insaturé, linéaire ou ramifié en $C_2$-$C_{10}$, de préférence en $C_2$-$C_6$, contenant de 2 à 4 fonctions alcools ; un monoalkylether en $C_1$-$C_3$, de préférence en $C_1$-$C_2$, d'un diol aliphatique saturé ou insaturé, linéaire ou ramifié en $C_2$-$C_{10}$, de préférence en $C_2$-$C_6$ ; un polyoxyalkylene glycol ou un monoether de polyoxyalkylene glycol, de formule $HO-[r-O]_nZ$ où Z représente de l'hydrogène ou un radical alkyle en $C_1$-$C_3$, de préférence en $C_1$-$C_2$, r un radical alkylene linéaire ou ramifié en $C_2$-$C_3$ et n va de 2 à 4, de préférence de 2 à 3 ; l'alcool benzylique ; le phénol, le biphenylediol -4, 4′, les bis (hydroxyphenyl) alcanes présentant une chaîne aliphatique en $C_1$-$C_3$.

A titre d'exemple, on peut citer : l'éthanol, le propanol, l'isopropanol, les butanols, le 2-éthylhexanol, alcool laurique, l'alcool cétylique ; le cyclohexanol ; l'alcool allylique, l'alcool méthallylique ; les butanediols, les hexanediols, le glycerol, le pentaérythritol ; les mono-, di-, tri-, et tetra-ethylene glycols, les mono-, di-, tri-, et tetra-propylene glycols, ainsi que leur methyl- ou ethyl- monoether ; le 2,2-bis(4 - hydroxyphenyl) propane.

Les catalyseurs pouvant être mis en oeuvre sont les catalyseurs classiques de transesterification choisis parmi les sels ioniques basiques du type hydrures, hydroxydes, alcoolates, acétates, carbonates, bicarbonates, acétylacétonates, ... de métaux alcalins.

On peut citer en particulier : la soude, la potasse, la lithine, le carbonate de sodium, de potassium ...

Parmi les composés complexants susceptibles de solubiliser au moins partiellement et de dissocier au moins partiellement le catalyseur de transesterification peuvent être mis en oeuvre :

- les agents séquestrants décrits dans le brevet français n° 2.450.120 et répondant à la formule :

$N-(CHR_1-CHR_2-O-(CHR_3-CHR_4-O)_n-R_5)_3$ (I)

dans laquelle n est un nombre entier supérieur ou égal à 0 et inférieur ou égal à 10 ($0 \leq n \leq 10$), $R_1$, $R_2$, $R_3$, et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $-C_mH_{2m}-O$ ou $C_mH_{2m+1}-O-$, où m est compr entre 1 et 12 ($1 \leq m \leq 12$).

- les polyethers macrocycliques appelés "ethers couronne" décrits dans le brevet français n° 2.026.481, lesdits polyethers macrocycliques ayant de 12 à 40 atomes dans le cycle et constitués de 4 à 10 unités -O-X dans lesquelles X est soit $-CHR_6-CHR_7-$ soit $-CHR_6-CHR_8-CR_9R_7-$, $R_6$, $R_7$, $R_8$, et $R_9$ identiques ou différents étant un atome d'hydrogène ou un radical alkyle ayant de 1 à 4 atomes de carbone, un des X pouvant être $-CHR_6-CHR_8-CR_9R_7-$ quand les unités -O-X comprennent le groupement $-O-CHR_6CHR_7-$.

- les composés macrocycliques ou bicycliques appelés "cryptants" décrits dans le brevet français n° 2.052.947 et répondant aux formules IIa et IIb

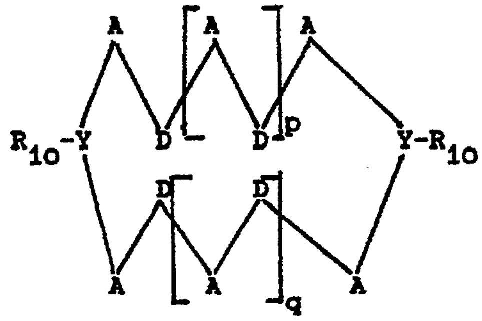

(IIa)

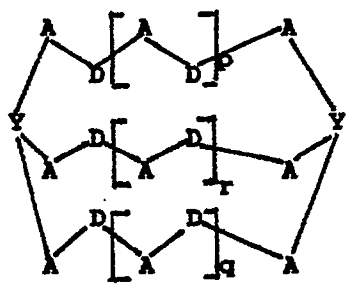

(IIb)

dans lesquelles :

- Y représente N ou P
- A représente un groupement alkylène ayant de 1 à 3 atomes de carbone
- D représente O, S ou N-$R_{11}$ où $R_{11}$ représente un radical alkyle ayant de 1 à 6 atomes de carbone
- $R_{10}$ représente un radical alkyle ayant de 1 à 6 atomes de carbone, p, q et r identiques ou différents sont des nombres entiers compris entre 1 et 5.
- les agents séquestrants supportés décrits dans le brevet européen n° 46706 caractérisés en ce qu'ils sont constitués par un support polymère organique réticulé et par une pluralité de groupes fonctionnels, fixés sur ledit support, de formule générale :

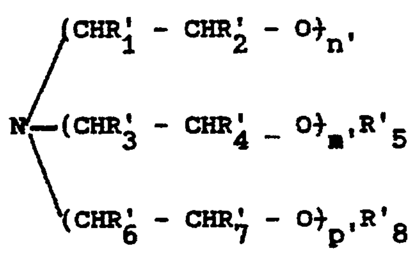

(III)

dans laquelle $R_1'$, $R_2'$, $R_3'$, $R_4'$, $R_6'$ et $R_7'$ identiques ou différents sont chacun un atome d'hydrogène ou jun radical alkyle ayant de 1 à 4 atomes de carbone, $R'_5$ et $R'_8$ identiques ou différents représentent un atome d'hydrogène, un radical alkyle ou cycloalkyle ayant de 1 à 12 atomes de carbone, un radical phényle, un radical $-C_{q'}H_{2q'}-\emptyset$ ou $C_{q'},H_{2q'}+1$-O-avec q' supérieur ou égal à 1 et inférieur ou égal à environ 12, et dans laquelle n', m', et p' identiques ou différents sont supérieurs ou égaux à 1 et inférieurs ou égaux à 10.

- les "éthers couronne" et les "cryptants" greffés sur des supports polymères organiques réticulés décrits dans l'article Angew. Chem. Int. Ed. Engl. 18, 421-429 (1979)
- les polyethyleneglycols décrits dans J. Phys. Chem 1972, 76, 2152 (U. TAKAKI, T.E. HOGEN ESCH, J. SMID) obtenus par oligomérisation de l'oxyde d'éthylène et présentant au moins 4 unités monomériques d'oxyde d'éthylène, ainsi que les alkyldiethers correspondants présentant des chaînes alkyles en $C_1$-$C_2$.

Lorsque le composé complexant est un agent séquestrant, celui-ci répondra de préférence à la formule (I) dans laquelle $R_1$, $R_2$, $R_3$ et $R_4$ représentent un atome d'hydrogène ou un radical méthyle, $R_5$ et n ayant la signification précédente.

Parmi ces derniers, on préfère encore plus particulièrement mettre en oeuvre les agents séquestrants pour lesquels n est supérieur ou égal à 0 et inférieur ou égal à 6 et pour lesquels $R_5$ représente un radical alkyle ayant de 1 à 4 atomes de carbone.

On peut citer :

- la tris(oxa-3 butyl)amine de formule :
$N-(CH_2-CH_2-O-CH_3)_3$
- la tris(dioxa-3,6 heptyl)amine de formule :
$N-(CH_2-CH_2-O-CH_2-CH_2-O-CH_3)_3$
- la tris(trioxa-3,6,9 décyl)amine de formule :
$N-(CH_2-CH_2-O-CH_2-CH_2-O-CH_2-CH_2-O-CH_3)_3$
- la tris(dioxa-3,6 octyl)amine de formule :
$N-(CH_2-CH_2-O-CH_2-CH_2-O-C_2H_5)_3$
- la tris(trioxa-3,6,9 undécyl)amine de formule :
$N-(CH_2-CH_2-O-CH_2-CH_2-O-CH_2-CH_2-O-C_2H_5)_3$
- la tris(dioxa-3,6 nonyl)amine de formule :
$N-(CH_2-CH_2-O-CH_2-CH_2-O-C_3H_7)_3$
- la tris(trioxa-3,6,9 dodécyl)amine de formule :
$N-(CH_2-CH_2-O-CH_2-CH_2-O-CH_2-CH_2-O-C_3H_7)_3$
- la tris(dioxa-3,6 décyl)amine de formule :
$N-(CH_2-CH_2-O-CH_2-CH_2-O-C_4H_9)_3$
- la tris(trioxa-3,6,9 tridécyl)amine de formule :
$N-(CH_2-CH_2-O-CH_2-CH_2-O-CH_2-CH_2-O-C_4H_9)_3$
- la tris (tetra-oxa-3,6,9,12 tridecyl) amine de formule :
$N-(CH_2-CH_2-O-(CH_2-CH_2-O)_3-CH_3)_3$
- la tris (hexa-oxa-3,6,9,12,15,18 nonadecyl)amine de formule :
$N-(CH_2-CH_2-O-(CH_2-CH_2-O)_5\ CH_3)_3$
- la tris(dioxa-3,6 méthyl-4 heptyl) amine de formule :
$N-(CH_2-CH_2-OCH-(CH_3)-CH_2-O-CH_3)_3$
- la tris(dioxa-3,6 diméthyl-2,4 heptyl) amine de formule :
$N-(CH_2-CH-(CH_3-OCH(CH3)-CH_2-O-CH_3)_3$.

On peut citer comme exemples d'éthers couronnes pouvant être utilisés selon l'invention :

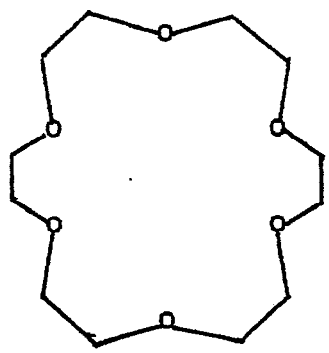

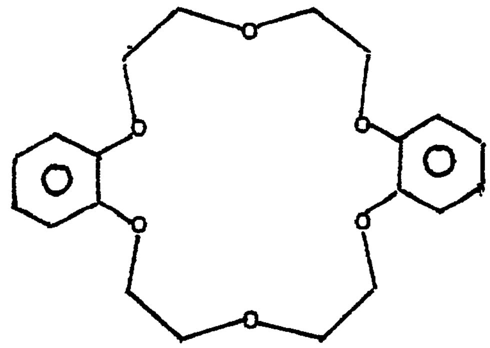

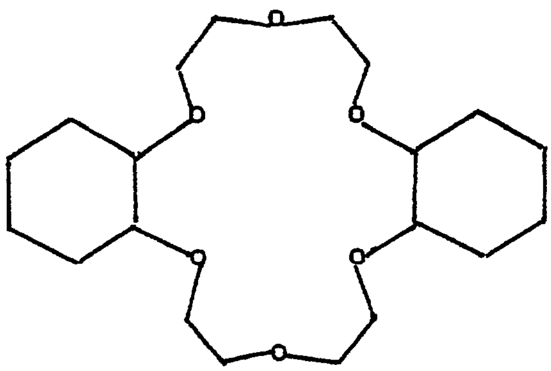

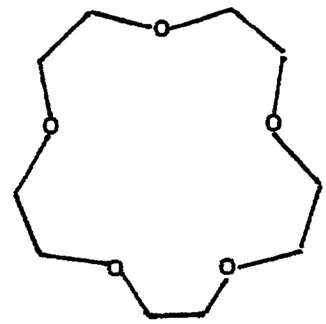

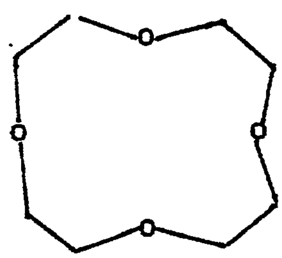

On peut citer comme exemples de composés macrocycliques ou bicycliques:

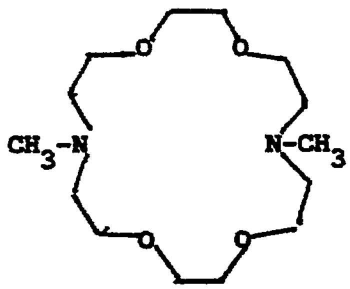

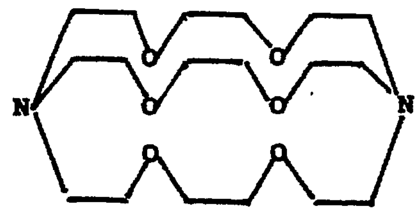

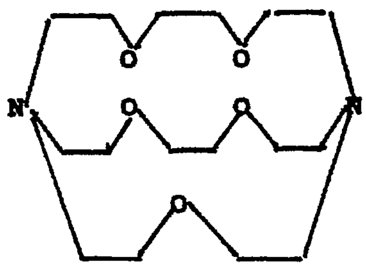

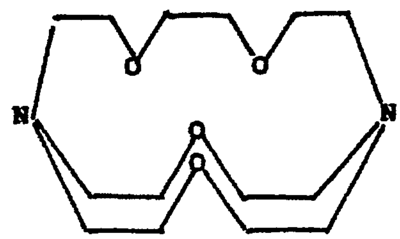

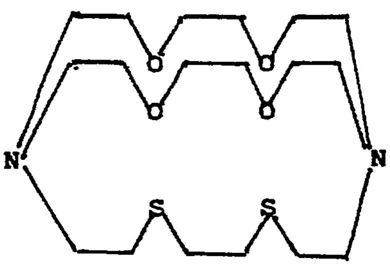

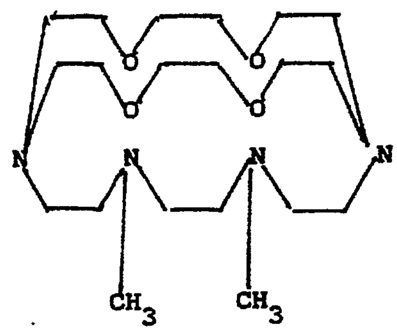

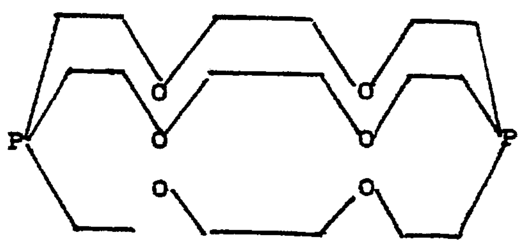

Lorsque le composé complexant est un agent séquestrant supporté, celui-ci est constitué par un support polymère organique réticulé et par une pluralité de groupes fonctionnels fixés sur ledit support, de formule générale (III) dans laquelle préférentiellement $R'_1$, $R'_2$, $R'_3$, $R'_4$, $R'_6$, et $R'_7$ représentent un atome d'hydrogène ou le radical méthyle et $R'_5$ et $R'_8$ représentent un atome d'hydrogène ou un radical alkyle ayant de 1 à 4 atomes de carbone, n', m', et p' étant supérieurs ou égaux à 1 et inférieurs ou égaux à 6.

On peut citer comme exemples de groupes fonctionnels, les groupes de formules suivantes :

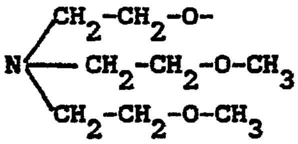

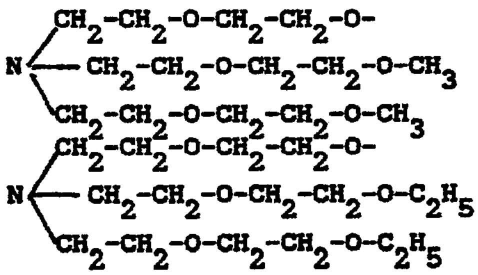

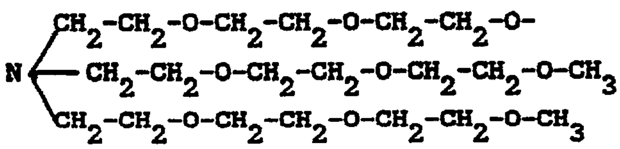

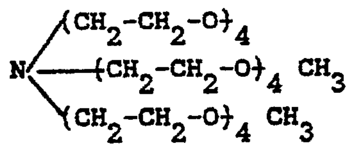

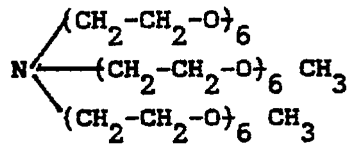

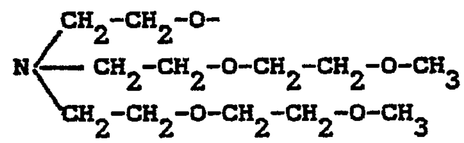

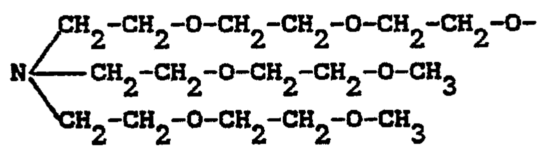

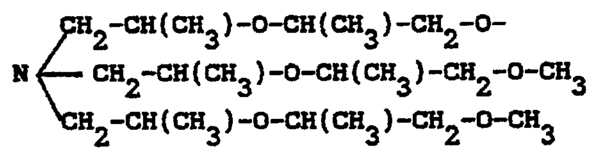

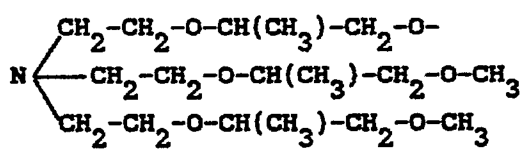

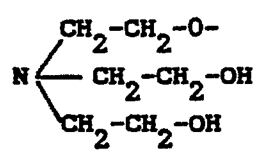

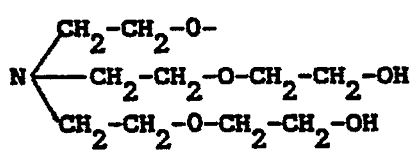

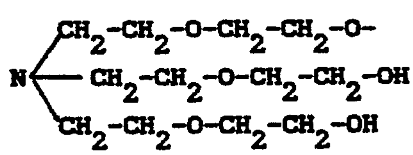

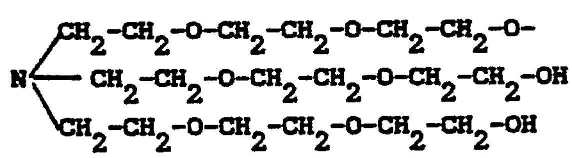

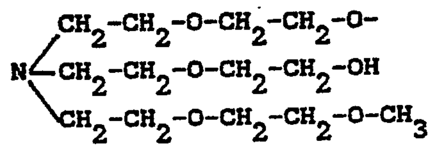

Le support peut dériver de tout polymère organique réticulé comportant des groupements substituables par les groupements fonctionnels de formule (III).

On peut citer comme exemples de polymères organiques adaptés à la présente invention, les polymères dérivés de composés vinylaromatiques tels que le styrène, le methylstyrène et les copolymères de composés vinylaromatiques et de diènes conjugués en $C_4$-$C_6$ tels que les copolymères du styrène et du butadiène et du styrène et de l'isoprène.

On préfère tout particulièrement utiliser comme polymère organique le polystyrène chloro au bromo méthyle, l'agent de réticulation étant alors, suivant un mode de réalisation préférentiel, le divinylbenzène.

Le taux de réticulation est un facteur important. Il est en effet nécessaire que les groupes fonctionnels de formule (III) greffés sur le polystyrène soient actifs. Dans ce but, il est nécessaire que le taux de réticulation ne soit pas trop important pour ne pas empêcher la pénétration des réactifs. On préfère utiliser un polystyrène dont le taux de réticulation par le divinylbenzène est inférieur à environ 10%. Encore plus préférentiellement, le taux de réticulation est inférieur à 5% environ.

Le groupement substituable est de préférence le chlore ou le brome du radical chloro ou bromo methyl -$CH_2Cl$ ou -$CH_2Br$ fixé sur le noyau benzénique du polystyrène.

On préfère tout particulièrement que le pourcentage de noyaux benzéniques du polystyrène portant un groupement fonctionnel soit supérieur à 5%. Encore plus préférentiellement, ce pourcentage est supérieur à 10%.

On peut représenter les agents séquestrants supportés préférés par la formule suivante :

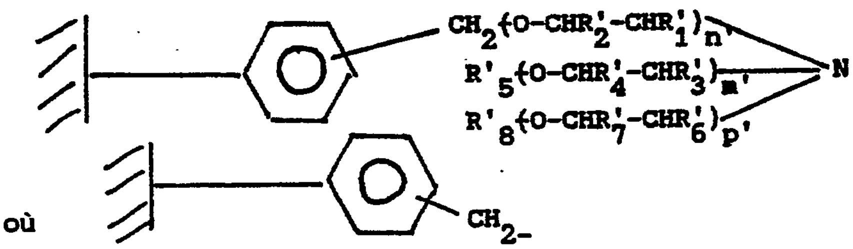

où

dérive du polystyrène chloro ou bromo méthylé réticulé par le divinylbenzène de formule :

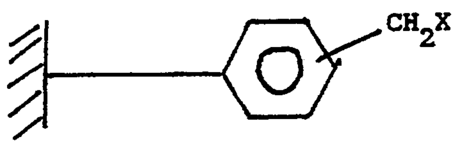

où X représente Cl ou Br.

Les "ethers couronne" et les "cryptants" greffés sur des supports organiques réticulés peuvent être obtenus par réaction d'un dérivé aminé approprié, d'un polyether macrocyclique ou d'un composé macrocyclique ou bicyclique avec un polystyrène chloromethylé. Les produits préférentiels peuvent être représentés par les formules suivantes :

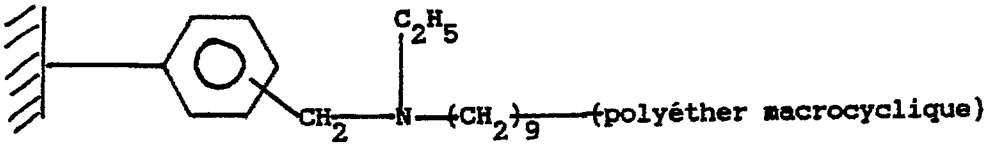

et

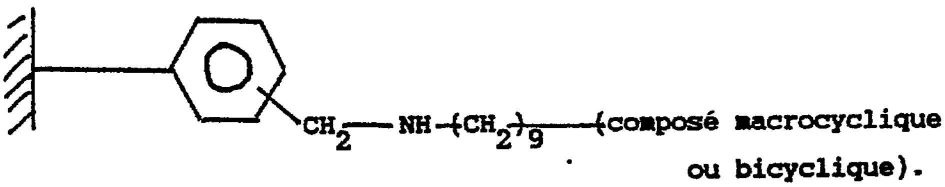

Pour une bonne mise en oeuvre du procédé faisant l'objet de l'invention, ladite opération de transesterification est réalisée en respectant les rapports suivants entre les différents réactifs :
- le rapport nombre de fonctions alcools/nombre de molécules-grammes de diester de l'acide carbonique dépend du nombre de fonctions esters (une ou deux) que l'on désire faire réagir ; la transesterification de deux fonctions esters sera bien entendu réalisée de préférence en présence d'un excès d'alcool par rapport à la stoechiometrie considérée ; celle d'une seule fonction ester sera bien évidemment réalisée de préférence en présence d'un excès de diester de l'acide carbonique par rapport à la stoechiometrie considérée. Ce rapport nombre de fonctions alcools/nombre de molécules-grammes de diester de l'acide carbonique est de l'ordre de 1/20 à 20/1, de préférence de l'ordre de 0,2 à 2,5
- le rapport molaire catalyseur/réactif en défaut (c'est-à-dire molécule-gramme de catalyseur/molécule-gramme de diester de l'acide carbonique dans le cas de la transestefification de deux fonctions ester, ou molécule-gramme de catalyseur/fonction alcool dans le cas de la transesterification d'une seule fonction ester) est de l'ordre de $10^{-4}$ à 5 $10^{-3}$, de préférence de l'ordre de 2 $10^{-4}$ à 2 $10^{-3}$,
- le rapport molaire composé complexant/catalyseur est de l'ordre de 0,001 à 0,1, de préférence de l'ordre de 0,003 à 0,1 et tout particulièrement de l'ordre de 0,004 à 0,03; ce rapport peut donc varier dans un large intervalle, mais il est préférable de ne pas utiliser une trop grande quantité de catalyseur basique notamment lorsque le diester et/ou l'alcool mis en oeuvre est (sont)insaturé (s), ce afin d'éviter la coloration du produit final.

Le procédé faisant l'objet de l'invention peut être réalisé en présence d'un composé "azéotropeur" susceptible de donner un azéotrope avec l'alcool ou les alcools formés par transesterification, afin de faciliter l'élimination dudit ou desdits alcools ; la nature dudit "azéotropeur" est bien entendu fonction de celle de l'alcool à éliminer ; l'homme de l'art peut sans difficulté trouver l'"azeotropeur" le mieux adapté ; on peut citer à titre d'exemple l'hexane, l'heptane, le cyclohexane, le cyclopentane, le toluène, le xylène ...

Pour la bonne réalisation de l'invention, ledit procédé peut être effectué à une pression voisine de la pression atmosphérique. De façon générale, afin d'éviter les phénomènes de prise de coloration et d'oxydation, il est réalisé de préférence sous atmosphère inerte, en particulier lorsque le carbonate à préparer présente un ou des groupes allyliques (pour éviter les réactions de polymérisation).

La température minimale dans la masse réactionnelle est bien entendu fonction de la cinétique de réaction ; elle doit être suffisante pour initier la réaction de transestérification et pour permettre l'élimination de l'alcool ou des alcools formés ou de leurs azéotropes, par distillation; cette température est généralement d'au moins 40°C.

La température maximale dans la masse réactionnelle, c'est-à-dire en fin de réaction, n'est pas critique lorsque l'alcool ou les alcools et le diester de l'acide carbonique mis en oeuvre sont saturés ; par contre, lorsque l'alcool ou les alcools et/ou le diester est (sont) insaturé (s) cette température maximale doit être inférieure à celle de formation de sous-produits pouvant apporter une coloration ; cette température maximale est généralement inférieure à 120°C.

La température des vapeurs est voisine de la température d'ébullition de l'alcool ou des alcools formés ou de leurs azéotropes afin de permettre le reflux de l'alcool ou des alcools formés ou de leurs azéotropes.

En fin de réaction, le réactif en excès formé peut être éliminé par exemple par distillation sous pression réduite.

La procédé faisant l'objet de l'invention est particulièrement bien adapté à la préparation du carbonate de diallyle et du bis (allylcarbonate) de diéthylène glycol.

Selon l'invention le carbonate de diallyle est obtenu par transesterification de l'alcool allylique et d'un carbonate de dialkyle présentant des radicaux alkyles linéaires ou ramifiés en $C_1$-$C_3$, en présence dudit catalyseur de transesterification et dudit agent complexant.

Pour sa bonne réalisation, l'opération de transesterification est effectuée sous atmosphère inerte, de

préférence en présence d'un agent azéotropeur tel que l'hexane à une température telle que la température dans la masse réactionnelle en fin d'opération ne dépasse pas 120°C et la température des vapeurs soit voisine du point d'ébullition de l'alcool formé ou de l'azéotrope formé pour permettre le reflux de l'alcool formé ou de son azéotrope formé.

Les quantités de réactifs pouvant être mises en oeuvre correspondent aux rapports molaires suivants :
- alcool allylique/diester allant de 2/1 à 20/1, de préférence de 2,2 à 2,5
- catalyseur/diester allant de $10^{-4}$ à 5 $10^{-3}$, de préférence de 2 $10^{-4}$ à 2 $10^{-3}$
- complexant/catalyseur allant de 0,001 à 0,1, de préférence de 0,003 à 0,1, et tout particulièrement de 0,004 à 0,03.

Selon l'invention, le bis (allylcarbonate) de diéthylène glycol est obtenu par transesterification du carbonate de diallyle et du diéthylene glycol en présence dudit catalyseur de transesterification et dudit agent complexant.

Pour sa bonne réalisation, l'opération est effectuée sous atmosphère inerte, éventuellement en présence d'un agent azéotropeur tel que le toluène ou le xylène, à une température telle que la température dans la masse réactionnelle en fin d'opération ne dépasse pas 120°C et la température des vapeurs soit voisine du point d'ébulli tion de l'alcool allylique ou éventuellement de l'azéotrope formé afin de permettre le reflux de l'alcool formé ou de son azéotrope.

Les quantiiés de réactifs préférentiellement mis en oeuvre correspondent aux rapports molaires suivants:
- diéthylene glycol/carbonate de diallyle allant de 1/40 à 1/2, de préférence de 1/10 à 1/4 (ou bien nombre de fonctions alcools/ moles de carbonate de diallyle allant de 1/20 à 1, de préférence de 0,2 à 0,5)
- catalyseur/diethylène glycol allant de 2 $10^{-4}$ à $10^{-2}$, de préférence de 4 $10^{-4}$ à 4 $10^{-3}$ (ou bien moles de catalyseur/fonctions alcools allant de $10^{-4}$ à 0,5 $10^{-2}$, de préférence de 2 $10^{-4}$ à 2 $10^{-3}$)
- complexant/catalyseur de l'ordre de 0,001 à 0,1, de préférence de l'ordre de 0,003 à 0,1, et tout particulièrement de l'ordre de 0,004 à 0,03.

Les carbonates organiques préparés selon le procédé de l'invention peuvent être utilisés comme cela est bien connu, comme solvants pour les composés cellulosiques, comme plastifiants... ; ceux qui sont insaturés peuvent être utilisés comme monomères pour la préparation de polymères à propriétés optiques.

Les exemples suivants sont donnés à titre indicatif et ne peuvent être considérés comme une limite du domaine et de l'esprit de l'invention.

Exemple 1

Dans un réacteur inerte de 300 litres maintenu à pression atmosphérique et équipé d'une colonne à distiller, de condenseurs et d'un décanteur azéotropique, on introduit sous atmosphère d'azote :
. 68 kg (soit 855 moles) de diméthylcarbonate (DMC)
. 103,5 kg (soit 1783 moles) d'alcool allylique, ce qui correspond à un rapport fonction alcool/mole de DMC de 2,36
. 30 kg d'hexane technique
. 21,2 g (soit 0,53 mole) de soude, ce qui correspond à un rapport molaire soude/DMC de 0,07 $10^{-2}$
. 1 g (soit 4,55 $10^{-3}$ mole de pentaoxa -1, 4, 7, 10, 13- cyclopentadécane (éther 15 - couronne - 5) ce qui correspond à un rapport molaire complexant/catalyseur de 8,6 $10^{-3}$.

La masse réactionnelle est mise sous agitation puis portée à une température permettant le reflux de l'hétéroazéotrope hexane-méthanol à la pression atmosphérique.

Après équilibrage de la colonne, on commence à soutirer le méthanol formé en veillant à ce que la température sensible ne dépasse pas 50°C au maximum ; cette opération de soutirage est réalisée jusqu'à ce que la composition du milieu réactionnel du réacteur suivie pas chromatographie en phase gazeuse soit la suivante :
. méthanol : traces
. DMC : traces
. rapport molaire pondéral carbonate de diallyle/carbonate de méthylallyle ≧ 15,5.

L'opération de soutirage du méthanol a duré 9 heures.

Les produits de la masse réactionnelle contenus dans le réacteur sont ensuite séparés par distillation fractionnée, d'abord sous pression atmosphérique puis sous pression réduite. Le rendement molaire en carbonate de diallyle basé sur le DMC est d'au moins 95 % ; celui-ci peut encore être amélioré par recyclage du carbonate de méthylallyle.

Exemple 2

On réalise l'opération de transesterification décrite à l'exemple 1 en l'absence d'agent complexant et en augmentant la quantité de soude. On constate qu'il faut 0,250 kg de soude (soit 6,25 moles) c'est-à-dire 11,8 fois plus qu'à l'exemple 1, pour arriver également en 9 heures à une composition semblable du milieu du réacteur en fin de soutirage du méthanol.

Exemple 3

On réalise l'opération de transesterification décrite à l'exemple 1 en mettant en oeuvre :
. comme catalyseur 63,5 g (soit 0,96 mole) de potasse, ce qui correspond à un rapport molaire potasse/DMC de 0,13 $10^{-2}$
. comme complexant 7,6 g (soit 28,7 $10^{-3}$ mole) d'hexaoxa -1, 4, 7, 10, 13, 16- cyclooctadécane (éther 18 - couronne -6), ce qui correspond à un rapport molaire complexant/catalyseur de 0,03.

On constate qu'il faut 9 heures de soutirage du méthanol pour aboutir à une composition du milieu du réacteur semblable à celle de l'exemple 1.

Exemple 4

On réalise l'opération décrite à l'exemple 3 en l'absence de complexant et en mettant en oeuvre 350g (soit 5,3 moles) de potasse (ce qui correspond à un rapport molaire catalyseur/DMS de 0,7 $10^{-2}$) au lieu de 63,5g de potasse.

Une composition du milieu du réacteur semblable à celle de l'exemple 1 est obtenue après 12 heures de soutirage du méthanol.

On constate que par rapport à l'exemple 3 la présence d'un agent complexant a permis de diminuer par 5,5 la quantité de catalyseur à utiliser, et de diminuer de 25% le temps de réaction.

Exemple 5

Dans un réacteur inerte de 2 litres maintenu sous atmosphère d'azote et équipé d'une colonne à distiller, on introduit :
. 1421,5 g (soit 10 moles) de carbonate de diallyle
. 106,1 g (soit 1 mole ou 2 fonctions OH) de diethylène glycol (DEG), ce qui correspond à un rapport fonction alcool/mole de carbonate de diallyle de 0,2
. 0,138 g (soit $10^{-3}$ mole) de carbonate de potassium, ce qui correspond à un rapport molaire catalyseur/DEG de $10^{-3}$ ou à un rapport mole de catalyseur/fonction OH de 0,5 $10^{-3}$
. 2,64 $10^{-3}$ g (soit $10^{-5}$ mole) d'éther-18-couronne-6, ce qui correspond à un rapport molaire complexant/catalyseur de 0,01.

La masse réactionnelle est placée sous pression réduite (entre 13332 Pa et 26664 Pa) et portée à une température de l'ordre de 100 à 120°C pour permettre la distillation de l'alcool allylique formé.

La réaction est terminée au bout de 3 heures après le début de la distillation de l'alcool allylique.

Le carbonate de potassium est neutralisé, puis l'excès de carbonate de diallyle est éliminé par distillation (pression finale inférieure à 1333 Pa et température maximum de 150°C).

On récupère en pied 260 g de produit fini (ce qui correspond à un rendement pondéral de 94,5 % sur la stoechiométrie de la réaction de formation de monomère pur) dont la couleur se situe entre 5 et 10 HAZEN (Norme AFNOR NF-T 20-605)

La composition dudit produit, déterminée par chromatographie par permeation de gel est la suivante :
. monomère 84,5 %
. dimère 14,0 %
. trimère 1,5 %.

Sa viscosité est de 13,8 $10^{-6}$ $m^2/s$ à 25°C.

Exemple 6

On répète l'opération décrite à l'exemple 5, en mettant en oeuvre le couple catalyseur/complexant suivant :
. comme catalyseur $10^{-3}$ mole d'hydroxyde de lithium au lieu de $10^{-3}$ mole de carbonate de potassium
. et comme complexant $10^{-5}$ mole d'éther -12-couronne-4.

Après 3 heures de réaction, le rendement en produit recherché exprimé comme précedemment est de 94 %.

La couleur du produit fini est entre 5 et 10 HAZEN.

La composition de celui-ci est la suivante :
. monomère 82,7 %
. dimère 15,4 %
. trimère 1,3 %.

Sa viscosité est de 14 $10^{-6}$ $m^2/s$ à 25°C.

Exemple 7

On répète l'opération décrite à l'exemple 5 en mettant en oeuvre le couple catalyseur/complexant suivant :
. comme catalyseur $10^{-3}$ mole de carbonate de potassium

. comme complexant $10^{-5}$ mole de tris(3, 6-dioxahepytl) amine, au lieu de $10^{-5}$ mole d'éther-18-couronne-6.

Après 3 heures de réaction, le rendement en produit recherché exprimé comme précedemment est de 94 %.

Ce produit présente la composition suivante :

. monomère 83,8 %
. dimère 14,5 %
. trimère 1,7 %.

Sa viscosité est de 13,9 $10^{-6} m^2/s$ à 25°C.

Exemple 8

On répète l'opération décrite à l'exemple 5 en mettant en oeuvre le couple catalyseur/complexant suivant :

. comme catalyseur $10^{-3}$ mole de carbonate de potassium
. comme complexant $10^{-5}$ mole d'hexaoxa 4, 7, 13, 16, 21, 24 diaza 1, 10 bicyclo 8, 8, 8 hexacosane (cryptate 2, 2, 2), au lieu de $10^{-5}$ mole d'éther -18-couronne-6.

Après 3 heures de réaction le rendement en produit recherché exprimé comme précedemment est de 94,5 %.

Le produit présente la composition suivante :

. monomère 84,7 %
. dimère 14 %
. trimère 1,3 %

Sa viscosité est de 13, 7 $10^{-6} m^2/s$ à 25°C.

Exemple 9

On répète l'opération décrite à l'exemple 5 en mettant en oeuvre le couple catalyseur/complexant suivant :

. comme catalyseur, 2, 3 $10^{-3}$ mole de potasse (au lieu de $10^{-3}$ mole de carbonate de potassium), ce qui représente un rapport molaire catalyseur/DEG de 2, 3 $10^{-3}$ ou un rapport mole de catalyseur/fonction OH de 1,15 $10^{-3}$
. comme complexant 11, 4 $10^{-6}$ mole (au lieu de $10^{-5}$ mole) d'éther-18-couronne 6, ce qui correspond à un rapport molaire complexant/catalyseur voisin de 5 $10^{-3}$.

Après 3 heures de réaction, le rendement en produit recherché exprimé comme précedemment est de 94,5 %.

Le produit présente la composition suivante :

. monomère 84,2
. dimère 14,2 %
. trimère 1,4 %

Sa viscosité est de 13,8 $10^{-6} m^2/s$ à 25°C.

Exemple 10-21

On répète l'opération décrite à l'exemple 5 en faisant varier les quantités de carbonate de potassium et d'éther-18-couronne 6.

Les résultats obtenus figurent au tableau I.

Exemple 22

On répète l'opération décrite à l'exemple 5 en suivant le même mode opératoire et en mettant en oeuvre les réactifs suivants :

. 12 moles de carbonate de diallyle
. 1 mole de DEG, ce qui correspond à un rapport fonction alcool/-mole de carbonate de diallyle de 0,17
. 1,1 $10^{-3}$ mole de carbonate de potassium, ce qui correspond à un rapport mole de catalyseur/fonction OH de 0,55 $10^{-3}$
. 30 $10^{-6}$ mole d'éther 18-couronne-6, ce qui correspond à un rapport molaire complexant/catalyseur de 0,027.

Après 3 heures de réaction, on récupère un produit présentant la composition suivante :

. monomère 87,0 %
. dimère 12,0 %
. trimère 1,0 %.

Exemple 23 : Préparation de dicyclohexylcarbonate

Dans un réacteur de 1 litre, on introduit sous atmosphère d'azote:

- 1 mole de carbonate d'allyle,
- $10^{-3}$ mole de potasse,
- $10^{-5}$ mole d'éther 18-couronne-6.

A 110°C, 2 moles de cyclohexanol sont introduites en 2 heures à l'aide d'une ampoule de coulée. Dès la fin de l'introduction de cet alcool, la masse réactionnelle est portée à 140°C et on poursuit la réaction pendant 4h30 mn. Durant ce temps, l'alcool allylique formé est éliminé du milieu par distillation. Après neutralisation de la potasse le pied réactionnel est filtré.

Par chromatographie en phase gazeuse on retrouve :
- allylcyclohexylcarbonate : 0,03 mole
- dicyclohexylcarbonate : 0,725 mole

Le rendement global sur les matières premières qui ont effectivement réagi est proche de 95 %.

Exemple 24 : Préparation de bisallylcarbonates de dipropylèneglycol

On répète l'opération décrite à l'exemple 5 en remplaçant le DEG (1 mole) par du dipropylène glycol (1 mol).

La composition du produit obtenu, déterminée par chromatographie par perméation de gel, est la suivante :

$CH_2 = CH - CH_2\ CO_3\ C_3H_6\ O\ C_3H_6\ CO_3\ CH_2 - CH = CH_2$ 86,3%

$CH_2 = CH - CH_2(\ CO_3\ C_3H_6\ O\ C_3H_6)_2\ CO_3\ CH_2 - CH = CH_2$ 12,5%

$CH_2 = CH - CH_2(\ CO_3\ C_3H_6\ O\ C_3H_6)_3\ CO_3\ CH_2 - CH = CH_2$ 1,2%

Le rendement compté sur le dipropylèneglycol est de 100 %.

Exemple 25 : Préparation de bis méthylcarbonates de tétraéthylèneglycol

Dans un réacteur de 1 l on introduit sous atmosphère d'azote :
- diméthylcarbonate : 10 moles
- potasse : $10^{-2}$ mole
- éther-18-couronne 6 : 1,1 $10^{-5}$ mole.

puis, on chauffe aux environs de 80°C. A l'aide d'une ampoule de coulée on ajoute alors en 40 mn 1 mole de tétraéthylèneglycol. La température de la masse est ensuitek portée progressivement à 98°C, celle de la tête de la colonne ne dépassant pas 60°C (ébullition de l'azéotrope méthanol-carbonate de méthyle). Au bout de 3 heures la distillation s'arrête. La potasse est neutralisée et l'excès de diméthylcarbonate distillé. La pied réactionnel est filtré à température ambiante. La composition déterminée par chromatographie par perméation de gel est la suivante :
- $CH_3\ CO_3\ (CH_2)_2\ O\ (CH_2)_2\ O\ (CH_2)_2\ O\ (CH_2)_2\ CO_3\ CH_3$ = 74 %
- $CH_3\ [CO_3\ (CH_2)_2\ O\ (CH_2)_2\ O\ (CH_2)_2\ O\ (CH_2)_2]_2\ CO_3\ CH_3$ = 22 %
- $CH_3\ [CO_3\ (CH_2)_2\ O\ (CH_2)_2\ O\ (CH_2)_2\ O\ (CH_2)_2]_3\ CO_3\ CH_3$ = 4 %

Le rendement compté sur le tétraéthylène glycolest de 100 %.

Exemple 26 : Préparation de carbonates de l'éthermonoéthylique du diéthylèneglycol

Dans un réacteur de 1 l on introduit sous atmosphère d'azote :
- éthermonoéthylique du diéthylèneglycol : 2 moles
- $K_2CO_3$ : 2,9 $10^{-3}$ mole
- éther-18 couronne 6 : 1,3 $10^{-5}$ mole
- diméthylcarbonate : 1,33 mole
- hexane : 5,25 moles (1.3 étant placé dans une ampoule de coulée pour compenser celui qui est distillé avec le méthanol formé).

La masse réactionnelle est portée à 60°C et ne doit pas dépasser 80°C.

Le distillat n'est soutiré que lorsque la température en tête de colonne est inférieure ou égale à 47°C (température d'ébullition de l'hétéroazéotrope hexane-méthanol). Lorsque le distillat ne présente plus qu'une seule phase on arrête la réaction. Le carbonate de potassium est neutralisé et l'excès de carbonate de méthyl éliminé par distillation.

Le produit fini filtré est analysé par chromatographie en phase gazeuse.

Les pourcentages en surfaces donnent :
- éthermonoéthylique du diéthylèneglycol : 0,28 mole
- $C_2H_5\ O\ C_2H_4\ O\ C_2H_4\ CO_3\ CH_3$ : 0,31 mole
- $C_2H_5\ O\ C_2H_4\ O\ C_2H_4\ CO_3\ C_2H_4\ O\ C_2H_4\ O\ C_2H_5$ : 0,70 mole

Le rendement global compté sur l'éther monoéthylique du diéthylèneglycol est de 99 %.

Exemple 27 : Préparation de carbonates du 2-éthyl 1-hexanol

Dans un réacteur de 1 litre, on introduit sous atmosphère d'azote:
- 2 éthyl-1-hexanol : 2 moles

- potasse : 3 $10^{-3}$ mole
- éther-18 couronne 6 : 7,5 $10^{-5}$ mole
- diméthylcarbonate : 1,33 mole
- hexane : 3,9 moles (1/3 étant placé dans l'ampoule de coulée).

L'essai est conduit comme à l'exemple 26.

L'analyse par chromatographie en phase gazeuse donne :

2 - éthyl 1 - hexanol :0,31 mole

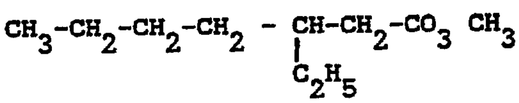 : 0,43 mole

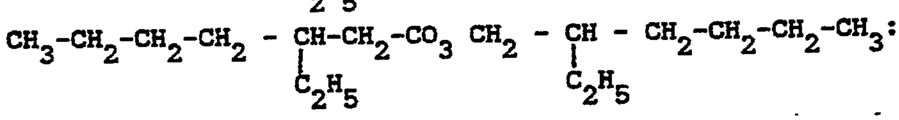 : 0,63 mole

Le rendement exprimé sur le 2-éthyl-1-hexanol qui a réagi est de 99%.

Exemple 28 : Préparation de carbonage cyclique du butanediol-1,2

Dans un réacteur de 1 litre, on introduit sous atmosphère d'azote:

1,2 butanediol : 1 mole

diméthylcarbonate : 2 moles

$K_2CO_3$ : 3,6 $10^{-3}$ mole

éther 18-couronne 6 : 1,3 $10^{-5}$ mole

hexane : 3,84 moles (dont 1/3 dans une ampoule de coulée).

Le processus opératoire est le même qu'à l'exemple 26. On obtient 0,95 mole de 1,3 -dioxolan -2 - one - 4 - éthyl :

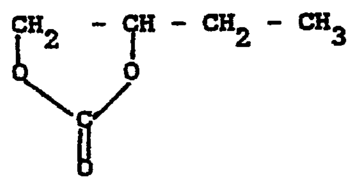

soit un rendement de 95 % sur le diol engagé.

**TABLEAU I**

| EX | $K_2CO_3$ en mole / $K_2CO_3$ / DEG molaire | Complexant en mole | Complexant / $K_2CO_3$ molaire | Composition du produit fini: mono % | di % | tri % | poly % |
|---|---|---|---|---|---|---|---|
| 10 | $0,5\ 10^{-3}$ | 0 | - | produit ayant l'aspect d'un gel | | | |
| 11 | $0,5\ 10^{-3}$ | $5,0\ 10^{-6}$ | 0,01 | 81,9 | 16,1 | 2,0 | - |
| 12 | $0,5\ 10^{-3}$ | $6,5\ 10^{-6}$ | 0,013 | 83,5 | 14,8 | 1,7 | - |
| 13 | $0,7\ 10^{-6}$ | $7\ 10^{-6}$ | 0,01 | 84,9 | 13,8 | 1,3 | - |
| 14 | $0,7\ 10^{-3}$ | $14\ 10^{-6}$ | 0,02 | 85,0 | 13,8 | 1,2 | - |
| 15 | $1,4\ 10^{-3}$ | $7\ 10^{-6}$ | 0,005 | 85,0 | 14,0 | 1,0 | - |
| 16 | $1,4\ 10^{-3}$ | $14\ 10^{-6}$ | 0,01 | 85,4 | 13,5 | 1,1 | - |
| 17 | $1,4\ 10^{-3}$ | $28\ 10^{-6}$ | 0,02 | 83,8 | 14,5 | 1,7 | - |
| 18 | $2,1\ 10^{-3}$ | $10,5\ 10^{-6}$ | 0,005 | 85,2 | 13,5 | 1,3 | - |
| 19 | $2,1\ 10^{-3}$ | $21\ 10^{-6}$ | 0,01 | 84,5 | 14,0 | 1,5 | - |
| 20 | $2,1\ 10^{-3}$ | $42\ 10^{-6}$ | 0,02 | 84,0 | 14,6 | 1,4 | - |
| 21 | $2,2\ 10^{-3}$ | $10\ 10^{-6}$ | 0,0045 | 84,6 | 14,0 | 1,4 | - |

"mono" signifie "monomère" "di" signifie "dimère" "tri" signifie "trimère " "poly" signifie "polymère"

**Revendications**

1) Procédé de préparation de carbonates organiques par transesterification d'un diester de l'acide carbonique et d'au moins un alcool plus lourd que celui ou ceux formés par transesterification, en présence d'un catalyseur de transesterification à base d'un sel ionique basique, caractérisé en ce que ladite opération de transesterification est réalisée en outre en présence d'un composé complexant susceptible de solubiliser au moins partiellement ledit sel dans le milieu réactionnel par complexation de son cation et de dissocier au moins partiellement ledit sel.

2) Procédé selon la revendication 1 caractérisé en ce que le composé complexant est un agent sequestrant de formule

$N-(CHR_1-CHR_2-O-(CHR_3-CHR_4-O)_n-R_5)_3$ (I)

dans laquelle n est un nombre entier supérieur ou égal à 0 et inférieur ou égal à 10 ($0 \leq n \leq 10$), $R_1$, $R_2$, $R_3$ et $R_4$ identiques ou différents représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical $-C_m H_{2m}-$Ø ou $C_m H_{2m+1}-$Ø-, où m e compris entre 1 et 12 ($1 \leq m \leq 12$).

3) Procédé selon la revendication 1 caractérisé en ce que l'agent complexant est un polyether macrocyclique "ether couronne" ayant de 12 à 40 atomes dans le cycle et constitués de 4 à 10 unités -O-X dans lesquelles X est soit $-CHR_6-CHR_7-$ soit $-CHR_6-CHR_8-CR_9R_7-$, $R_6$, $R_7$, $R_8$, et $R_9$ identiques ou différents étant un atome d'hydrogène ou un radical alkyle ayant de 1 à 4 atomes de carbone, un des X pouvant être $-CHR_6-CHR_8-CR_9R_7-$ quand les unités -O-X comprennent le groupement $-O-CHR_6-CHR_7-$.

4) Procédé selon la revendication 1 caractérisé en ce que l'agent complexant est un composé macrocyclique ou bicyclique "cryptant" répondant aux formules IIa et IIb

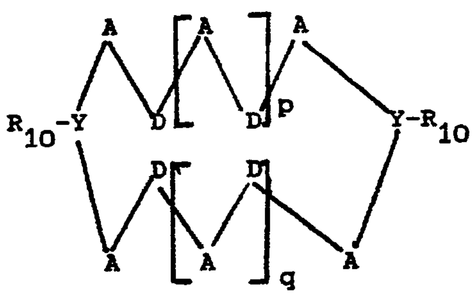

(IIa)

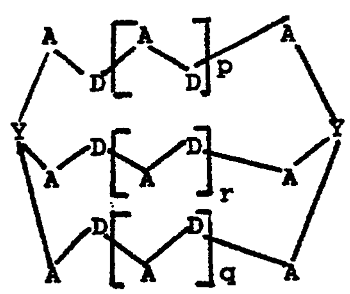

(IIb)

dans lesquelles :

- Y représente N ou P
- A représente un groupement alkylène ayant de 1 à 3 atomes de carbone
- D représente O, S ou $N-R_{11}$ où $R_{11}$ représente un radical alkyle ayant de 1 à 6 atomes de carbone
- $R_{10}$ représente un radical alkyle ayant de 1 à 6 atomes de carbone, p, q et r identiques ou différents sont des nombres entiers compris entre 1 et 5.

5) Procédé selon la revendication 1 caractérisé en ce que l'agent complexant est un agent séquestrant supporté constitué par un support polymère organique réticulé et par une pluralité de groupes fonctionnels, fixés sur ledit support, de formule générale :

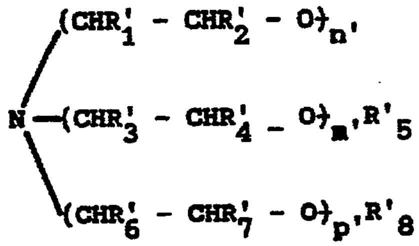

(III)

dans laquelle $R_1'$, $R_2'$, $R_3'$, $R_4'$, $R_6'$, et $R_7'$ identiques ou différents sont chacun un atome d'hydrogène ou un radical alkyle ayant de 1 à 4 atomes de carbone, $R_5'$ et $R_8'$ identiques ou différents représentent un atome d'hydrogène, un radical alkyle ou cycloalkyle ayant de 1 à 12 atomes de carbone, un radical phényle, un radical $-C_{q'}H_{2q'}-\emptyset$ ou $C_{q'}H_{2q'+1}-O-$avec q' supérieur ou égal à 1 et inférieur ou égal à environ 12, et dans laquelle n', m', et p' identiques ou différents sont supérieurs ou égaux à 1 et inférieurs ou égaux à 10.

6) Procédé selon la revendication 1 caractérisé en ce que l'agent complexant est un "éther-couronne" greffé sur des supports polymères organiques réticulés.

7) Procédé selon la revendication 1 caractérisé en ce que l'agent complexant est un "cryptant" gréffé sur des supports polymères organiques réticulés.

8) Procédé selon la revendication 1 caractérisé en ce que l'agent complexant est un polyethyleneglycol obtenu par oligomérisation de l'oxyde d'éthylène et présentant au moins 4 unités monomériques d'oxyde d'éthylène, ou un alkyldiether correspondant présentant des chaînes alkyles en $C_1$-$C_2$.

9) Procédé selon l'une quelconque des revendications précédents caractérisé en ce que le diester de l'acide carbonique a pour formule

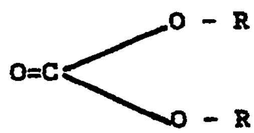

où R et R' sont identiques ou différents et représentent des radicaux alkyles linéaires ou ramifiés en $C_1$-$C_{10}$, ou alkenykes linéaires ou ramifiés en $C_3$-$C_{10}$.

10) Procédé selon la revendication 9) caractérisé en ce que R et R' sont identiques ou différents et représentent des radicaux alkyles linéaires ou ramifiés en $C_1$-$C_3$ ou alkenyles linéaires ou ramifiés en $C_3$-$C_4$.

11) Procédé selon l'une quelconque des revendications précédentes caractérisé en ce que l'alcool est un monoalcool aliphatique saturé ou insaturé, linéaire ou ramifié en $C_2$-$C_{22}$ ; un monoalcool cycloaliphatique en $C_6$ ; un polyol aliphatique saturé ou insaturé, linéaire ou ramifié en $C_2$-$C_{10}$ contenant de 2 à 4 fonctions alcools ; un monoalkylether en $C_1$-$C_3$ d'un diol aliphatique saturé ou insaturé, linéaire ou ramifié en $C_2$-$C_{10}$ ; un polyoxyalkylene glycol ou un monoether de polyoxyalkylene glycol, de formule $HO\!-\!(r\!-\!O)_n\!-\!Z$ où Z représente de l'hydrogène ou un radical alkyle en $C_1$-$C_3$, r un radical alkylene linéaire ou ramifié en $C_2$-$C_3$ et n va de 2 à 4 ; l'alcool benzylique ; le phénol, le biphenylediol -4, 4', les bis (hydroxyphenyl) alcanes présentant une chaîne aliphatique en $C_1$-$C_3$.

12) Procédé selon l'une quelconque des revendications précédentes caractérisé en ce que le catalyseur est un hydrure, hydroxyde, alcoolate, acétate, carbonate, bicarbonate ou acétylacétonate de métaux alcalins.

13) Procédé selon la revendication 12) caractérisé en ce que le catalyseur est de la soude, de la potasse, de la lithine, du carbonate de sodium, de potassium.

14) Procédé selon l'une quelconque des revendications précédentes caractérisé en ce que l'opération de transesterification est réalisée en respectant les rapports suivants entre les différents réactifs :
- nombre de fonctions alcools/nombre de molécules-grammes de diester de l'acide carbonique de l'ordre de 1/20 à 20/1
- rapport molaire catalyseur/réactif en défaut de l'ordre de $10^{-4}$ à $5 \cdot 10^{-3}$
- rapport molaire composé complexant/catalyseur de l'ordre de 0,001 à 0,1.

15) Procédé selon la revendication 14 cartactérisé en ce que les rapports entre les différents réactifs sont les suivants :
- nombre de fonction alcools/nombre de molécules-grammes de diester de l'acide carbonique de l'ordre de 0,2 à 2,5
- rapport molaire catalyseur/réactif en défaut de l'ordre de $2 \cdot 10^{-4}$ à $2 \cdot 10^{-3}$
- rapport molaire composé complexant/catalyseur de l'ordre de 0,044 à 0,03.

16) Procédé selon l'une quelconque des revendications précédentes caractérisé en ce que ladite opération de transesterificiation est réalisée en présence d'un composé azéotropeur de l'alcool formé ou des alcools formés.

17) Procédé selon l'une quelconque des revendications précédentes caractérisé en ce que la températuture de la masse réactionnelle est telle qu'elle permet d'initier la réaction de transestérification et d'éliminer par distillation l'alcool ou les alcools formés ou leurs azéotropes, et en ce que la température des vapeurs est voisin de la température d'ébullition de l'alcool formé ou des alcools formés ou de leurs azéotropes.

18) Procédé selon la revendication 17 caractérisé en ce que la température de la masse réactionnelle est d'au moins 40°C et est inférieure à 120°C.

19) Procédé selon l'une quelconque des revendications précédentes caractérisé en ce qu'un carbonate de dialkyle présentant des radicaux alkyles linéaires ou ramifiés en $C_1$–$C_3$ est transesterifié sous atmosphère inerte en carbonate de diallyle à l'aide d'alcool allylique, les quantités de réactifs mises en oeuvre correspondant aux rapports molaires suivants :
- alcool allylique/carbonate de dialkyle allant de 2/1 à 20/1
- catalyseur/carbonate de dialkyle allant de $10^{-4}$ à $5{\cdot}10^{-3}$
- complexant/catalyseur allant de 0,001 à 0,1.

20) Procédé selon la revendication 19 caractérisé en ce que les quantités de réactifs mises en oeuvre correspondent aux rapports molaires suivants :
- alcool allylique/carbonate de dialkyle allant de 2,2 à 2,5
- catalyseur/carbonate de dialkyle allant de $2{\cdot}10^{-4}$ à $2{\cdot}10^{-3}$
- complexant/catalyseur allant de 0,004 à 0,03.

21) Procédé selon l'une quelconque des revendications 1 à 18 caractérisé en ce que du carbonate de diallyle est transesterifié sous atmosphère inerte en bis(allylcarbonate) de diethylène glycol à l'aide de diethyleneglycol, les quantités de réactifs mises en oeuvre correspondant aux rapports molaires suivants :
- diethylene glycol/carbonate de diallyle allant de 1/40 à 1/2
- catalyseur/diethylène glycol allant de $2{\cdot}10^{-4}$ à $10^{-2}$
- complexant/catalyseur allant de 0,003 à 0,1.

22) Procédé selon la revendication 21 caractérisé en ce que les quantités de réactifs mises en oeuvre correspondent aux rapports molaires suivants :
- diethylene glycol/carbonate de diallyle allant de 1/10 à 1/4
- catalyseur/diethylène glycol allant de $4{\cdot}10^{-4}$ à $4{\cdot}10^{-3}$
- complexant/catalyseur allant de 0,004 à 0,03.

**Patentansprüche**

1. Verfahren zur Herstellung von organischen Carbonaten durch Umesterung eines Esters der Kohlensäure und zumindest eines Alkohols, der schwerer ist als der oder die Alkohole, die durch die Umesterung gebildet werden, in Gegenwart eines Umesterungskatalysators auf Basis eines basichen ionischen Salzes, dadurch gekennzeichnet, daß das Umesterungsverfahren außerdem in Gegenwart einer komplexierenden Verbindung durchgeführt wird, die in der Lage ist, das Salz zumindest teilweise in dem Reaktionsmilieu durch Komplexierung seines Kations zu solubilisieren und das Salz zumindest teilweise zu dissoziieren.

2. Verfahren gemäß Anspruch 1, dadurch gekennzeichnet, daß die komplexierende Verbindung ein Sequestriermittel der Formel

$$N\{CHR_1-CHR_2-O-(CHR_3-CHR_4-O)_n-R_5)_3 \qquad (I)$$

ist, worin n eine ganze Zahl größer oder gleich 0 ist und niedriger oder gleich 10 ist ($0 \le n \le 10$), $R_1$, $R_2$, $R_3$ und $R_4$, die gleich oder voneinander verschieden sind, für ein Wasserstoffatom oder einen Alkylrest mit 1 bis 4 Kohlenstoffatomen stehen, und $R_5$ einen Alkyl- oder Cycloalkylrest mit 1 bis 12 Kohlenstoffatomen, einen Phenylrest oder einen Rest $-C_mH_{2m}-Ø$ oder Rest $C_mH_{2m+1}-Ø-$ bedeutet, worin m zwischen 1 und 12 liegt ($1 \le m \le 12$).

3. Verfahren gemäß Anspruch 1, dadurch gekennzeichnet, daß das Komplexierungsmittel ein makrocyclischer Polyether in Form eines "Kronenethers" mit 12 bis 40 Kohlenstoffatomen in dem Ring ist, bestehend aus 4 bis 10 Einheiten $-O-X$, worin X für $-CHR_6-CHR_7-$ oder für $-CHR_6-CHR_8-CR_9R_7-$ steht, $R_6$, $R_7$, $R_8$ und $R_9$, die gleich oder voneinander verschieden sind, ein Wasserstoffatom oder einen Alkylrest mit 1 bis 4 Kohlenstoffatomen bedeuten, wobei einer der Reste X für $-CHR_6-CHR_8-CR_9R_7-$ stehen kann, wenn die Einheiten $-O-X-$ die Gruppe $-O-CHR_6-CHR_7-$ umfassen.

4. Verfahren gemäß Anspruch 1, dadurch gekennzeichnet, daß das Komplexierungsmittel eine makrocyclische oder bicyclische "Kryptand"-Verbindung der Formeln IIa und IIb

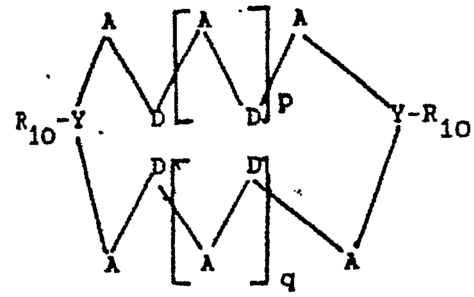

(IIa)

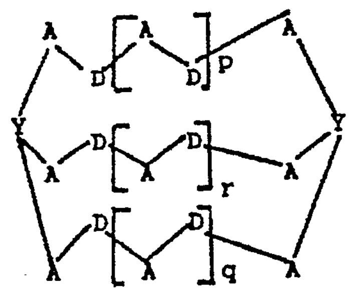

(IIb)

ist, worin
– Y für N oder P steht
– A für eine Alkylengruppe mit 1 bis 3 Kohlenstoffatomen steht,
– D O, S oder $N-R_{11}$ bedeutet, worin $R_{11}$ für einen Alkylrest mit 1 bis 6 Kohlenstoffatomen steht
– $R_{10}$ einen Alkylrest mit 1 bis 6 Kohlenstoffatomen bedeutet, p, q und r, die gleich oder voneinander verschieden sind, ganze Zahlen zwischen 1 und 5 sind.

5. Verfahren gemäß Anspruch 1, dadurch gekennzeichnet, daß das Komplexierungsmittel ein auf einen Träger aufgebrachtes Sequestriermittel ist, bestehend aus einem vernetzten organischen Polymerenträger und aus einer Vielzahl an diesen Träger gebundener funktioneller Gruppen der allgemeinen Formel

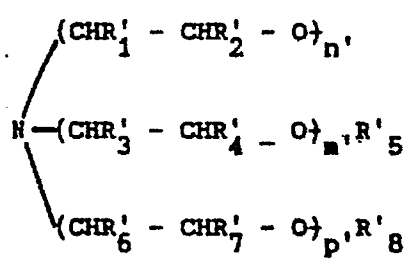

(III)

worin $R'_1$, $R'_2$, $R'_3$, $R'_4$, $R'_6$ und $R'_7$, die gleich oder voneinander verschieden sind, jeweils für ein Wasserstoffatom oder einen Alkylrest mit 1 bis 4 Kohlenstoffatomen stehen, $R'_5$und $R'_8$, die gleich oder voneinander verschieden sind, für ein Wasserstoffatom, einen Alkyl- oder Cycloalkylrest mit 1 bis 12 Kohlenstoffatomen, einen Phenylrest, einen Rest $-C_{q'}H_{2q'}-Ø$ oder $-C_{q'}H_{2q'+1}-O-$ stehen, worin q' grösser oder gleich 1 ist und niedriger oder gleich etwa 12 ist, und worin n', m' und p', die gleich oder voneinander verschieden sind, grösser oder gleich 1 und niedriger oder gleich 10 sind.

6. Verfahren gemäß Anspruch 1, dadurch gekennzeichnet, daß das Komplexiermittel ein auf die vernetzten organischen polymeren Träger gepfropfter "Kronenether" ist.

7. Verfahren gemäß Anspruch 1, dadurch gekennzeichnet, daß das Komplexiermittel ein auf die vernetzten organischen polymeren Träger gepfropfter "Kryptand" ist.

8. Verfahren gemäß Anspruch 1, dadurch gekennzeichnet, daß das Komplexiermittel ein durch Oligomerisation von Ethylenoxid erhaltenes Polyethylenglykol mit zumindest 4 monomeren Ethylenoxideinheiten oder ein entsprechender Alkyldiether mit $C_1-C_2$-Alkylketten ist.

9. Verfahren gemäß einem der vorhergehenden Ansprüche, dadurch gekennzeichnet, daß der Diester der Kohlensäure die Formel

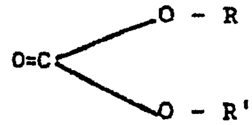

besitzt, worin R und R′, die gleich oder voneinander verschieden sind, lineare oder verzweigte $C_1$–$C_{10}$-Alkylreste oder lineare oder verzweigte $C_3$–$C_{10}$-Alkenylreste bedeuten.

10. Verfahren gemäß Anspruch 9, dadurch gekennzeichnet, daß R und R′, die gleich oder voneinander verschieden sind, lineare oder verzweigte $C_1$–$C_3$-Alkylreste oder lineare oder verzweigte $C_3$–$C_4$-Alkenylreste bedeuten.

11. Verfahren gemäß einem der vorhergehenden Ansprüche, dadurch gekennzeichnet, daß der Alkohol ein linearer oder verzweigter, gesättigter oder ungesättigter aliphatischer $C_2$–$C_{22}$-Monoalkohol; ein cycloaliphatischer $C_6$-Monoalkohol; ein lineares oder verzweigtes, gesättigtes oder ungesättigtes aliphatisches $C_2$–$C_{10}$-Polyol mit 2 bis 4 Alkoholfunktionen; ein $C_1$–$C_3$-Monoalkylether eines linearen oder verzweigten, gesättigten oder ungesättigten aliphatischen $C_2$–$C_{10}$-Diols; ein Polyoxyalkylenglykol oder ein Monoether eines Polyoxyalkylenglykols der Formel $HO{-}[R{-}O]_n{-}Z$, worin Z für Wasserstoff oder einen $C_1$–$C_3$-Alkylrest, R für einen linearen oder verzweigten $C_2$–$C_3$-Alkylenrest und n für eine Zahl von 2 bis 4 steht; Benzylalkohol; Phenol; 4,4′-Biphenyldiol; die Bis-hydroxy-phenylalkane mit einer aliphatischen $C_1$–$C_3$-Kette ist.

12. Verfahren gemäß einem der vorhergehenden Ansprüche, dadurch gekennzeichnet, daß der Katalysator ein Hydrid, Hydroxid, Alkoholat, Acetat, Carbonat, Bicarbonat oder Acetylacetonat der Alkalimetalle ist.

13. Verfahren gemäß Anspruch 12, dadurch gekennzeichnet, daß der Katalysator Natronlauge, Kalilauge, Lithiumhydroxid, Natriumcarbonat oder Kaliumcarbonat ist.

14. Verfahren gemäß einem der vorhergehenden Ansprüche, dadurch gekennzeichnet, daß das Umesterungsverfahren unter Beachtung der folgenden Verhältnisse zwischen den verschiedenen Reagenzien durchgeführt wird:
– Anzahl der Alkoholfunktionen/Anzahl der Grammoleküle des Diesters der Kohlensäure in der Größenordnung von 1:20 bis 20:1
– molares Verhältnis von Katalysator/Reagenz im Unterschuß in der Größenordnung von $10^{-4}$ bis $5 \cdot 10^{-3}$
– molares Verhältnis komplexierende Verbindung/Katalysator in der Größenordnung von 0,001 bis 0,1.

15. Verfahren gemäß Anspruch 14, dadurch gekennzeichnet, daß die Verhältnisse zwischen den verschiedenen Reagenzien die folgenden sind:
– Anzahl der Alkoholfunktionen/Anzahl der Grammoleküle des Diesters der Kohlensäure in der Größenordnung von 0,2 bis 2,5
– molares Verhältnis Katalysator/Reagenz im Unterschuß in der Größenordnung von $2 \cdot 10^{-4}$ bis $2 \cdot 10^{-3}$
– molares Verhältnis komplexierende Verbindung/Katalysator in der Größenordnung von 0,004 bis 0,03.

16. Verfahren gemäß einem der vorhergehenden Ansprüche, dadurch gekennzeichnet, daß das Umesterungsverfahren in Gegenwart einer azeotropierenden Verbindung des gebildeten Alkohols oder der gebildeten Alkohole durchgeführt wird.

17. Verfahren gemäß einem der vorhergehenden Ansprüche, dadurch gekennzeichnet, daß die Temperatur der Reaktionsmasse derart ist, daß sie es ermöglicht, die Umesterungsreaktion zu initiieren und den oder die gebildeten Alkohole oder deren Azeotrope durch Destillation zu eliminieren, und daß die Temperatur der Dämpfe in der Nähe der Siedetemperatur des gebildeten Alkohols oder der gebildeten Alkohole oder von deren Azeotropen liegt.

18. Verfahren gemäß Anspruch 17, dadurch gekennzeichnet, daß die Temperatur der Reaktionsmasse zumindest 40°C und weniger als 120°C beträgt.

19. Verfahren gemäß einem der vorhergehenden Ansprüche, dadurch gekennzeichnet, daß ein Dialkylcarbonat mit linearen oder verzweigten $C_1$–$C_3$-Alkylresten unter inerter Atmosphäre in Diallylcarbonat mit Hilfe des Allylalkohols durch Umesterung übergeführt wird, wobei die eingesetzten Mengen der Reagenzien den folgenden molaren Verhältnissen entsprechen:
– Allylalkohol/Dialkylcarbonat von 2:1 bis 20:1
Katalysator/Dialkylcarbonat von $10^{-4}$ bis $5 \cdot 10^{-3}$
– Komplexiermittel/Katalysator von 0,001 bis 0,1.

20. Verfahren gemäß Anspruch 19, dadurch gekennzeichnet, daß die eingesetzten Mengen der Reagenzien den folgenden molaren Verhältnissen entsprechen:
– Allylalkohol/Dialkylcarbonat von 2,2 bis 2,5
– Katalysator/Dialkylcarbonat von $2 \cdot 10^{-4}$ bis $2 \cdot 10^{-3}$
– Komplexiermittel/Katalysator von 0,004 bis 0,03.

21. Verfahren gemäß einem der Ansprüche 1 bis 18, dadurch gekennzeichnet, daß das Diallylcarbonat unter inerter Atmosphäre durch Umesterung in das Bis-(allylcarbonat) des Diethylenglykols mit Hilfe von Diethylenglykol übergeführt wird, wobei die Mengen der eingesetzten Reagenzien den folgenden molaren Verhältnissen entsprechen:
- Diethylenglykol/Diallylcarbonat von 1:40 bis 1:2
- Katalysator/Diethylenglykol von $2 \cdot 10^{-4}$ bis $10^{-2}$
- Komplexiermittel/Katalysator von 0,003 bis 0,1.

22. Verfahren gemäß Anspruch 21, dadurch gekennzeichnet, daß die eingesetzten Mengen der Reagenzien den folgenden molaren Verhältnissen entsprechen:
- Diethylenglykol/Diallylcarbonat von 1:10 bis 1:4
- Katalysator/Diethylenglykol von $4 \cdot 10^{-4}$ bis $4 \cdot 10^{-3}$
- Komplexiermittel/Katalysator von 0,004 bis 0,03.

## Claims

1. Process for the preparation of organic carbonates by transesterification of a diester of carbonic acid and of at least one alcohol heavier than that or those formed by transesterification, in the presence of a transesterification catalyst based on a basic ionic salt, characterized in that the said transesterification operation is additionally carried out in the presence of a complexing compound capable of at least partially solubilizing the said salt in the reaction mixture by complexing its cation and at least partially dissociating the said salt.

2. Process according to Claim 1, characterized in that the complexing compound is a sequestering agent of formula

$N\{CHR_1-CHR_2-O-(CHR_3-CHR_4-O)_n-R_5)_3$ (I )

in which n is an integer greater than or equal to 0 and smaller than or equal to 10 ($0 \leq n \leq 10$), $R_1$, $R_2$, $R_3$ and $R_4$, which are identical or different, denote a hydrogen atom or an alkyl radical containing from 1 to 4 carbon atoms, and $R_5$ denotes an alkyl or cycloalkyl radical containing from 1 to 12 carbon atoms, a phenyl radical or a radical $-C_mH_{2m}-Ø$ or $C_mH_{2m+1}-Ø-$, where m is between 1 and 12 ($1 \leq m \leq 12$).

3. Process according to Claim 1, characterized in that the complexing agent is "crown ether" macrocyclic polyether containing from 12 to 40 atoms in the ring and consisting of 4 to 10 $-O-X$ units in which X is either $-CHR_6-CHR_7-$ or $-CHR_6-CHR_8-CR_9R_7-$, $R_6$, $R_7$, $R_8$ and $R_9$, which are identical or different, being a hydrogen atom or an alkyl radical containing from 1 to 4 carbon atoms, it being possible for one of the X to be $-CHR_6-CHR_8-CR_9R_7-$ when the $-O-X$ units include the group $-O-CHR_6-CHR_7-$.

4. Process according to Claim 1, characterized in that the complexing agent is a "cryptant" macrocyclic or bicyclic compound corresponding to formulae IIa and IIb

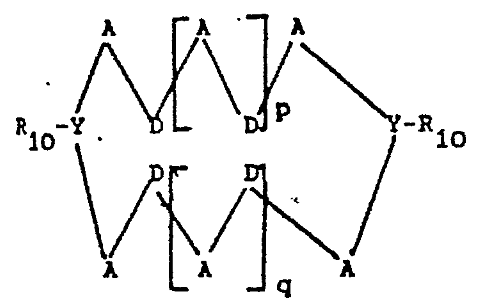

(IIa)

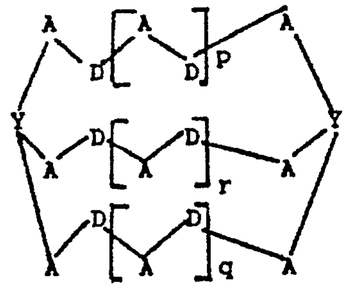

(IIb)

in which:
- Y denotes N or P
- A denotes an alkylene group containing from 1 to 3 carbon atoms

– D denotes O, S or N–$R_{11}$, where $R_{11}$ denotes an alkyl radical containing from 1 to 6 carbon atoms
– $R_{10}$ denotes an alkyl radical containing from 1 to 6 carbon atoms, and p, q and r, which are identical or different, are integers between 1 and 5.

5. Process according to Claim 1, characterized in that the complexing agent is a supported sequestering agent consisting of a crosslinked organic polymeric support and of a plurality of functional groups attached to the said support, of general formula:

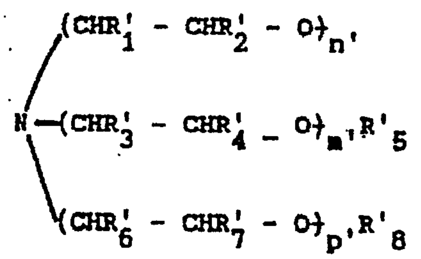

(III)

in which each of $R'_1$, $R'_2$, $R'_3$, $R'_4$, $R'_6$ und $R'_7$, which are identical or different, are a hydrogen atom or an alkyl radical containing 1 to 4 carbon atoms, $R'_5$ and $R'_8$, which are identical or different, denote a hydrogen atom, an alkyl or cycloalkyl radical containing from 1 to 12 carbon atoms, a phenyl radical or a radical $-C_{q'}H_{2q'}-Ø$ oder $-C_{q'}H_{2q'+1}-O-$ with q' greater than or equal to 1 and smaller than or equal to approximately 12, an in which n', m' und p', which are identical or different, are greater than or equal to 1 and smaller than or equal to 10.

6. Process according to Claim 1, characterized in that the complexing agent is a "crown ether" grafted onto crosslinked organic polymeric supports.

7. Process according to claim 1, characterized in that the complexing agent is a "cryptant" grafted onto crosslinked organic polymeric supports.

8. Process according to Claim 1, characterized in that the complexing agent is a polyethylene glycol obtained by oligomerization of ethylene oxide and containing at least 4 ethylene oxide monomeric units, or a corresponding alkyl diether containing $C_1$–$C_2$ alkyl chains.

9. Process according to any one of the preceding claims, characterized in that the carbonic acid diester has the formula

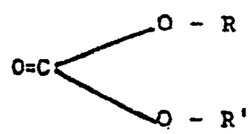

where R and R' are identical or different and denote linear or branched $C_1$–$C_{10}$ alkyl or linear or branched $C_3$–$C_{10}$ alkenyl radicals.

10. Process according to Claim 9, characterized in that R and R' are identical or different and denote linear or branched $C_1$–$C_3$ alkyl or linear or branched $C_3$–$C_4$ alkenyl radicals.

11. Process according to any one of the preceding claims, characterized in that the alcohol is a saturated or unsaturated, linear or branched $C_2$–$C_{22}$ aliphatic monoalcohol, a cycloaliphatic $C_6$ monoalcohol, a saturated or unsaturated, linear or branched $C_2$–$C_{10}$ aliphatic polyol containing from 2 to 4 alcohol functional groups, a $C_1$–$C_3$ monoalkyl ether of a saturated or unsaturated, linear or branched $C_2$–$C_{10}$ aliphatic diol, a polyoxyalkylene glycol or a polyxyalkylene glycol monoether, of formula $HO[r-O]_nZ$ where Z denotes hydrogen or a $C_1$–$C_3$ alkyl radical, r a linear or branched $C_2$–$C_3$ alkylene radical and n ranges from 2 to 4, benzyl alcohol, phenol, 4,4'-biphenyldiol, and bis(hydroxyphenol)alkanes containing a $C_1$–$C_3$ aliphatic chain.

12. Process according to any one of the preceding claims, characterized in that the catalyst is an alkali metal hydride, hydroxide, alcoholate, acetate, carbonate, bicarbonate or acetylacetonate.

13. Process according to Claim 12, characterized in that the catalyst is sodium hydroxide, potassium hydroxide, lithium hydroxide or sodium or potassium carbonate.

14. Process according to any one of the preceding claims, characterized in that the transesterification operation is carried out by conforming to the following ratios of the various reactants:
– numbre of alcohol functional groups/number of grammolecules of carbonic acid diester of the order of $1/20$ to $20/1$
– catalyst/deficient reactant molar ratio of the order of $10^{-4}$ to $5 \times 10^{-3}$
– complexing compounds/catalyst molar ratio of the order of 0.001 to 0.1.

15. Process according to Claim 14, characterized in that the ratios of the various reactants are as follows:
– number of alcohol functional groups/number of grammolecules of carbonic acid diester of the order of 0.2 to 2.5,
– catalyst/deficient reactant molar ratio of the order of $2\times10^{-4}$ to $2\times10^{-3}$
– complexing compound/catalyst molar ratio of the order of 0.004 to 0.03.

16. Process according to any one of the preceding claims characterized in that the said transesterification operation is carried out in the presence of a compound forming an azeotrope with the alcohol formed or the alcohols formed.

17. Process according to any one of the preceding claims, characterized in that the temperature of the reaction mass is such that it makes it possible to initiate the transesterification reaction and to remove the alcohol or the alcohols formed or their azeotropes by distillation, and in that the vapour temperature is close to the boiling temperature of the alcohol formed or of the alcohols formed or of their azeotropes.

18. Process according to Claim 17, characterized in that the temperature of the reaction mass is at least 40°C and is lower than 120°C.

19. Process according to any one of the preceding claims, characterized in that a dialkyl carbonate containing linear or branched $C_1$–$C_3$ alkyl radicals is transesterified under inert atmosphere to diallyl carbonate with the aid of allyl alcohol, the quantities of reactants used corresponding to the following molar ratios:
– allyl alcohol/dialkyl carbonate ranging from 2:1 to 20:1
– catalyst/dialkyl carbonate ranging from $10^{-4}$ to $5\times10^{-3}$

20. Process according to Claim 19, characterized in that the quantities of reactants used correspond to the following molar ratios:
– allyl alcohol/dialkyl carbonate ranging from 2.2 to 2.5
– catalyst/dialkyl carbonate ranging from $2\times10^{-4}$ to $2\times10^{-3}$
– complexant/catalyst ranging from 0.004 to 0.03.

21. Process according to any one of Claims 1 to 18, characterized in that diallyl carbonate is transesterified under inert atmosphere to diethylene glycol bis(allyl carbonate) with the aid of diethylene glycol, the quantities of reactants used corresponding to the following molar ratios:
– diethylene glycol/diallyl carbonate ranging from $1/40$ to $1/2$
– catalyst/diethylene glycol ranging from $2\times10^{-4}$ to $10^{-2}$
– complexant/catalyst ranging from 0.003 to 0.1.

22. Process according to Claim 21, characterized in that the quantities of reactants used correspond to the following molar ratios:
– diethylene glycol/diallyl carbonate ranging from $1/10$ to $1/4$
– catalyst/diethylene glycol ranging from $4\times10^{-4}$ to $4\times10^{-3}$
– complexant/catalyst ranging from 0.004 to 0.03.